# EUROPÄISCHE PATENTSCHRIFT

(11) **EP 1 179 335 B1**
(45) Veröffentlichungstag und Bekanntmachung des Hinweises auf die Patenterteilung: **03.11.2004**
(21) Anmeldenummer: 01118396.9
(22) Anmeldetag: 28.07.2001
(51) Int. Cl.: A61K 7/06, C11D 1/52

(54) **Verwendung propoxylierter C10-C18-Fettsäurealkanolamide in Haarwaschmitteln**
Use of propoxylated C10-C18 fatty acid alkanolamides in shampoos
Utilisation d'alcanolamides d'acides gras C10-C18 propoxylées dans des shampooings

(30) Priorität: 12.08.2000 DE 10039742
(43) Veröffentlichungstag der Anmeldung: 13.02.2002
(73) Patentinhaber: KPSS-Kao Professional Salon Services GmbH, 64297 Darmstadt (DE)
(72) Erfinder: Heinz, Dieter, 65462 Gustavsburg (DE)

(56) Entgegenhaltungen:
- EP-A- 0 074 264
- WO-A-96/20993
- WO-A-98/25579
- WO-A-99/46356
- DATABASE WPI Week 199710 Derwent Publications Ltd., London, GB; AN 1997-103672 XP002139619 & JP 08 337560 A (KAWAKEN FINE CHEM CO LTD), 24. Dezember 1996 (1996-12-24)

## Beschreibung

Die Erfindung betrifft die Verwendung von propoxylierten C₁₀-C₁₈-Fettsäurealkanolamiden in konditionierenden Haarwaschmitteln.

Von einem guten Haarwaschmittel erwartet der Verbraucher vor allem eine zufriedenstellende Reinigungswirkung, eine gute Schaumentwicklung bei der Anwendung, sparsamen Verbrauch, gute Haut- und Schleimhautverträglichkeit ohne jedwede hautreizende oder allergisierende Wirkung und zumeist auch einen haarkonditionierenden Effekt.

Es sind zahlreiche Shampoos bekannt, die sich bemühen, diesen Anforderungen gerecht zu werden, ohne dies allerdings immer zu erreichen.

Dokument WO- A- 98 25579 offenbart Haarwaschmittel, in denen eine konditionierende Wirkung durch die Verwendung von kationischen Ammoniumverbindungen in Kombination mit Guerbet Alkoholen erreicht wird.

Dokument WO-A -99 46356 offenbart kosmetische Zubereitungen enthaltend propoxylierte Fettsäurealkanolamide.

Die Erfindung stellt nun ein Shampoo zur Verfügung, das die obengenannten Voraussetzungen erfüllt.
Das erfindungsgemäße Haarwaschmittel wird dadurch erhalten, daß einer wäßrigen Grundlage, enthaltend 5 bis 50 Gew.-%, bezogen auf die Gesamtzusammensetzung, mindestens eines anionischen, amphoteren, zwitterionischen und/oder nichtionischen Tensids, ein propoxyliertes C₁₀-C₁₈-Fettsäurealkanolamid zugesetzt wird.

Bei diesem Fettsäurealkanolamid handelt es sich vorzugsweise um eines der allgemeinen Formel 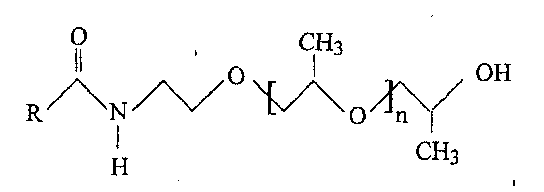 worin R einer C₁₀-C₁₈-Alkylgruppe und n eine Zahl von 1 bis 3 darstellt.
Der Anteil dieses Fettsäurealkanolamids in dem Haarwaschmittel liegt vorzugsweise bei 0,25 bis 10, insbesondere 0,5 bis 5 Gew.-%, berechnet auf die Gesamtzusammensetzung des Haarwaschmittels.

Die Verwendung dieser Fettsäurealkanolamide als Schaumstabilisator und Verdickungsmittel in Körperreinigungsmittel ist an sich bereits bekannt; es war jedoch für den Fachmann überraschend und nicht vorhersehbar, daß dadurch eine hervorragende haarkonditionierende Wirkung in Haarwaschmitteln erzielt werden kann.

Diese Shampoo-Zusammensetzungen enthalten die in Shampoos üblichen Stoffe.

Solche sind insbesondere Tenside, bevorzugt anionische Tenside, in einer Menge von 5 bis 50 Gew.-%, vorzugsweise 10 bis 25 Gew.-% der Zusammensetzung.

Geeignete anionaktive Tenside im Rahmen der Erfindung sind solche vom Sulfat-, Sulfonat-, Carboxylat- und Alkylphosphat-Typ, vor allem natürlich diejenigen, die in Shampoos üblicherweise zum Einsatz gelangen, beispielsweise die bekannten C₁₀-C₁₈-Alkylsulfate und insbesondere die entsprechenden Ethersulfate, beispielsweise C₁₂-C₁₄-Alkylethersulfat, Laurylethersulfat, insbesondere mit 1 bis 4 Ethylenoxidgruppen im Molekül, weiterhin Monoglycerid(ether)sulfate, Fettsäureamidsulfate, die durch Ethoxylierung und anschließende Sulfatierung von Fettsäurealkanolamiden erhalten werden, und deren Alkalisalze sowie Salze langkettige Mono- und Dialkylphosphate, die milde, hautverträgliche Detergentien darstellen.

Im Rahmen der Erfindung weiterhin geeignete anionische Tenside sind α-Olefinsulfonate bzw. deren Salze und insbesondere Alkalisalze von Sulfobernsteinsäurehalbestern, beispielsweise das Dinatriumsalz des Monooctylsulfosuccinats und Alkalisalze langkettiger Monoalkylethoxysulfosuccinate.

Geeignete Tenside vom Carboxylat-Typ sind Alkylpolyethercarbonsäuren und deren Salze der Formel

R - (C₂H₄O)ₙ - O - CH₂ COOX,

worin R eine C₈-C₂₀-Alkylgruppe, vorzugsweise eine C₁₂-C₁₄-Alkylgruppe, n eine Zahl von 1 bis 20, vorzugsweise 2 bis 17, und X H oder vorzugsweise ein Kation der Gruppe Natrium, Kalium, Magnesium und Ammonium, das gegebenenfalls hydroxyalkylsubstituiert sein kann, bedeuten, sowie Alkylamidopolyethercarbonsäuren der allgemeinen Formel 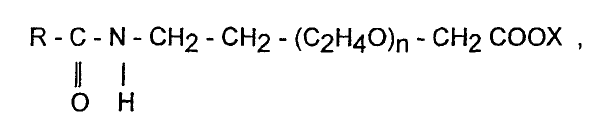 worin R und X die vorstehend angegebene Bedeutung haben, und n insbesondere für eine Zahl von 1 bis 10, vorzugsweise 2,5 bis 5, steht.

Derartige Produkte sind seit längerem bekannt und im Handel, beispielsweise unter den Handelsnamen "AKYPO® " und "AKYPO-SOFT® ".

Auch C₈-C₂₀-Acylisethionate können, allein oder im Gemisch mit anderen Tensiden, eingesetzt werden, ebenso Sulfofettsäuren und deren Ester.
Es können auch Mischungen aus mehreren anionischen Tensiden eingesetzt werden, beispielsweise ein Gemisch aus einem α-Olefinsulfonat und einem Sulfosuccinat, vorzugsweise im Verhältnis von 1 : 3 bis 3 : 1, oder einem Ethersulfat und einer Polyethercarbonsäure oder Alkylamidoethercarbonsäure.

Eine Übersicht über die in flüssigen Körperreinigungsmitteln zum Einsatz gelangenden anionaktiven Tenside findet sich im übrigen in der Monographie von K.Schrader, "Grundlagen und Rezepturen der Kosmetika", 2. Aufl. (1989, Hüthig Buchverlag), S. 595-600 und S. 683 bis 691.

Der besonders bevorzugte Mengenbereich an anionischen Tensiden in den erfindungsgemäß eingesetzten flüssigen Shampoos liegt zwischen 5 und 35 Gew.-%, insbesondere bei 7,5 bis 25 Gew.-%, besonders bevorzugt bei 10 bis 20 Gew.-%, berechnet auf die Gesamtzusammensetzung des Mittels.

Weitere geeignete anionische Tenside sind auch C₈-C₂₂-Acylaminocarbonsäuren bzw. deren wasserlösliche Salze, vorzugsweise in einer Menge von 0,5 bis 10, insbesondere 1 bis 7,5 Gew.-%, berechnet auf die Gesamtzusammensetzung. Besonders bevorzugt sind N-Lauroylglutamat, insbesondere als Natriumsalz, sowie beispielsweise N-Lauroylsarcosinat, N-C₁₂-C₁₈-Acylasparaginsäure, N-Myristoylsarcosinat, N-Oleoylsarcosinat, N-Lauroylmethylalanin, N-Lauroyllysin und N-Lauroylaminopropylglycin, vorzugsweise in Form ihrer wasserlöslichen Alkali- oder Ammonium-, insbesondere Natriumsalze, vorzugsweise im Gemisch mit den obengenannten anionaktiven Tensiden.

Weitere geeignete Tenside in den erfindungsgemäß eingesetzten Haarwaschmitteln sind nichtionische Tenside, vorzugsweise im Gemisch mit anionaktiven und/oder zwitterionischen bzw. amphoteren Tensiden.
Diese sind bei Schrader, I.c., auf den Seiten 600-601 und S. 694-695 beschrieben. Geeignet sind insbesondere Alkylpolyglucoside mit der allgemeinen Formel

R - O - (R¹O)ₙ-Zₓ,

worin R eine Alkylgruppe mit 8 bis 18 Kohlenstoffatomen, R¹ eine Ethylen- oder Propylengruppe, Z einen Saccharidrest mit 5 bis 6 Kohlenstoffatomen, n eine Zahl von 0 bis 10 und x eine Zahl zwischen 1 und 5 bedeuten.
Diese Alkylpolyglucoside sind in letzter Zeit insbesondere als ausgezeichnete hautverträgliche schaumverbessernde Mittel in flüssigen Wasch- und Körperreinigungsmitteln bekannt geworden und sind in einer Menge von 1 bis 20, insbesondere 2,5 bis 10 Gew.-%, der Gesamtzusammensetzung enthalten.

Gemische aus anionaktiven Tensiden und Alkylpolyglucosiden sowie deren Verwendung in flüssigen Körperreinigungsmitteln sind an sich bereits bekannt, beispielsweise aus der EP-A 70 074. Die dort beschriebenen Alkylpolyglucoside sind prinzipiell auch im Rahmen der vorliegenden Erfindung geeignet; ebenso die aus der EP-A 358 216 bekannten Gemische aus Sulfosuccinaten und Alkylpolyglucosiden.

Weitere nichtionische Tensidbestandteile sind beispielsweise langkettige Fettsäuremono- und -dialkanolamide, wie Cocosfettsäuremonoethanolamid und Myristinfettsäuremonoethanolamid, die auch als Schaumverstärker eingesetzt werden können. Andere zusätzlich mitverwendbare nichtionische Tenside sind z.B. die verschiedenen Sorbitanester, wie Polyethylenglykolsorbitanstearinsäureester, Fettsäurepolyglykolester oder auch Mischkondensate aus Ethylenoxid und Propylenoxid, wie sie beispielsweise unter der Handelsbezeichnung "Pluronics" im Verkehr sind. Weitere zusätzlich einsetzbare Tenside sind Aminoxide in einer Menge von 0,25 bis 5, vorzugsweise 0,5 bis 3,5 Gew.-%, berechnet auf die Gesamtzusammensetzung des Mittels.

Solche Aminoxide gehören seit langem zum Stand der Technik, beispielsweise C₁₂-C₁₈-Alkyldimethylaminoxide wie Lauryldimethylaminoxid, C₁₂-C₁₈-Alkylamidopropyl- oder -ethylaminoxide, C₁₂-C₁₈-Alkyldi(hydroxyethyl)- oder -(hydroxypropyl)aminoxide, oder auch Aminoxide mit Ethylenoxid- und /oder Propylenoxidgruppen in der Alkylkette.
Solche Aminoxide sind beispielsweise unter den Bezeichnungen "Ammonyx® ", "Aromox® " oder "Genaminox® " im Handel.
Die erfindungsgemäß eingesetzten Zusammensetzungen können als weiteren Tensid-Bestandteil amphotere bzw. zwitterionische Tenside, beispielsweise in einer Menge von 0,5 bis 10, vorzugsweise von 1 bis 7,5 Gew.-%, bezogen auf die Gesamtzusammensetzung, enthalten, ebenfalls vorzugsweise im Gemisch mit anionischen und/oder nichtionischen Tensiden.

Als solche sind insbesondere die verschiedenen bekannten Betaine wie Fettsäureamidoalkylbetaine und Sulfobetaine, beispielsweise Laurylhydroxysulfobetain, zu nennen; auch langkettige Alkylaminosäuren wie Cocoaminoacetat, Cocoaminopropionat und Natriumcocoamphopropionat und -acetat haben sich als geeignet erwiesen.
Im einzelnen können Betaine der Struktur 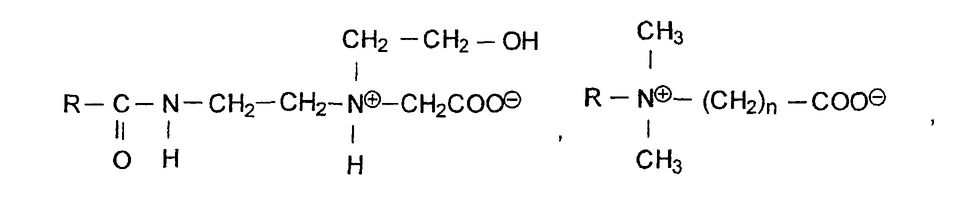 wobei R eine C₈-C₁₈-Alkylgruppe und n 1 bis 3 bedeuten,
Sulfobetaine der Struktur 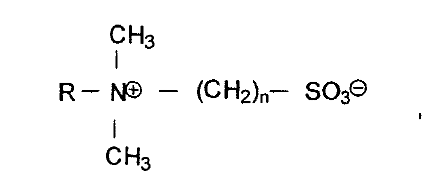 wobei R eine C₈-C₁₈-Alkylgruppe und n 1 bis 3 bedeuten,
und Amidoalkylbetaine der Struktur 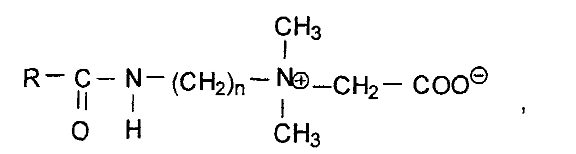 wobei R eine C₈-C₁₈-Alkylgruppe und n 1 bis 3 bedeuten, verwendet werden.

Die erfindungsgemäß eingesetzten Shampoos können neben den propoxylierten Fettsäurealkanolamiden auch konditionierende Wirkstoffe wie Eiweißhydrolysate und Polypeptide, z.B., Keratinhydrolysate, Kollagenhydrolysate vom Typ "Nutrilan^{R}" oder Elastinhydrolysate sowie insbesondere auch pflanzliche, gegebenenfalls kationisierte Eiweißhydrolysate, z.B. "Gluadin^{R}", enthalten.

Weitere mögliche Zusätze sind haarkonditionierende Polymere. Diese können nichtionische Polymere, vorzugsweise alkohol- und/oder wasserlösliche Vinylpyrrolidon-Polymere wie ein Vinylpyrrolidon-Homopolymerisat oder -Copolymerisat, insbesondere mit Vinylacetat, sein.
Geeignete Vinylpyrrolidon-Polymere sind z.B. die unter dem Handelsnamen "Luviskol®" bekannten Produkte, beispielsweise die Homopolymerisate "Luviskol® K 30, K 60 und K 90" sowie die wasser- bzw. alkohollöslichen Copolymerisate aus Vinylpyrrolidon und Vinylacetat, die unter dem Handelsnamen "Luviskol® VA 55 bzw. VA 64" von der BASF AG vertrieben werden.
Weitere geeignete nichtionische Polymere sind Vinylpyrrolidon/Vinylacetat/Vinylpropionat-Copolymere wie "Luviskol® VAP 343", Vinylpyrrolidon/(Meth)Acrylsäureester-Copolymere sowie Chitosan-Derivate. Ihr Anteil in den erfindungsgemäßen Haarwaschmitteln liegt, wenn vorhanden, zwischen 0,05 und 5, vorzugsweise 0,1 und 2,5, insbesondere 0,15 bis 1,5 Gew.-%, bezogen auf die Gesamtzusammensetzung des Mittels.

Anstelle oder zusätzlich zu den nichtionischen Polymeren können als haarkonditionierende Polymere auch kationische und/oder anionische und/oder amphotere Polymere in den genannten Mengen eingesetzt werden.

Bevorzugte haarkonditionierende kationische Polymere sind die altbekannten quaternären Cellulosederivate des Typs "Polymer JR®" sowie quaternisierte Homo- und Copolymere des Dimethyldiallylammoniumchlorids, wie sie unter dem Handelsnamen "Merquat®" im Handel sind, quaternäre Vinylpyrrolidon-Copolymere, insbesondere mit Dialkylaminoalkyl(meth)acrylaten, wie sie unter dem Namen "Gafquat®" bekannt sind, Copolymerisate aus Vinylpyrrolidon und Vinylimidazoliniummethochlorid, die unter dem Handelsnamen "Luviquat®" angeboten werden, Polyamino-Polyamid-Derivate, beispielsweise Copolymere von Adipinsäure-Dimethylaminohydroxypropyldiethylentriamin, wie sie unter dem Namen "Cartaretine® F" vertrieben werden, sowie auch bisquaternäre langkettige Ammoniumverbindungen der in der US-PS 4 157 388 beschriebenen Harnstoff-Struktur, die unter dem Handelsnamen "Mirapol® A 15" im Handel sind.

Verwiesen wird in diesem Zusammenhang auch auf die in den DE-OSen 25 21 960, 28 11 010, 30 44 738 und 32 17 059 genannten kationaktiven Polymeren sowie die in der EP-A 337 354 auf den Seiten 3 bis 7 beschriebenen Produkte. Es können auch Mischungen verschiedener kationischer Polymerer eingesetzt werden.

Zu den kationischen Polymeren zählen auch die in der EP-A 524 612 und der EP-A 640 643 beschriebenen Quaternisierungsprodukte aus Pfropfpolymerisaten von Organopolysiloxanen und Polyethyloxazolinen.

Als amphotere Polymere, die allein oder im Gemisch mit mindestens einem weiteren kationischen, nichtionischen oder anionischen Polymeren zum Einsatz gelangen, seien insbesondere Copolymerisate aus N-Octylacrylamid, (Meth)Acrylsäure und tert.-Butylaminoethylmethacrylat vom Typ "Amphomer®"; Copolymerisate aus Methacryloylethylbetain und Alkylmethacrylaten vom Typ "Yukaformer®", z. B. das Butylmethacrylat-Copolymere "Yukaformer® Am75"; Copolymerisate aus Carboxylgruppen und Sulfongruppen enthaltenden Monomeren, z. B. (Meth)Acrylsäure und Itaconsäure, mit basischen Gruppen, insbesondere Aminogruppen, enthaltenden Monomeren wie Mono- bzw. Dialkylaminoalkyl(meth)acrylaten bzw. Mono- bzw. Dialkylaminoalkyl(meth)acrylamiden; Copolymere aus N-Octylacrylamid, Methylmethacrylat, Hydroxypropylmethacrylat, N-tert.-Butylaminoethylmethacrylat und Acrylsäure sowie die aus der USA 3,927,199 bekannten Copolymeren genannt.

Geeignete anionische Polymere sind Vinylalkylether-, insbesondere Methylvinylether/Maleinsäure-Copolymere, die durch Hydrolyse von Vinylether/Maleinsäureanhydrid-Copolymeren entstehen und unter der Handelsbezeichnung "Gantrez® AN oder ES" vertrieben werden. Diese Polymeren können auch teilverestert sein, beispielsweise "Gantrez® ES 225", der Ethylester eines Ethylvinylethers/Maleinsäure-Copolymers, oder der Butyl- oder Isobutylester desselben.

Weitere geeignete anionische Polymere sind insbesondere Vinylacetat/Crotonsäureoder Vinylacetat/Vinylneodecanoat/Crotonsäure-Copolymere des Typs "Resyn®"; Natriumacrylat/Vinylalkohol-Copolymere des Typs "Hydagen® F", Natriumpolystyrolsulfonat, z.B. "Flexan® 130"; Ethylacrylat/Acrylsäure/N-tert.-Butylacrylamid-Copolymere des Typs "Ultrahold®"; Vinylpyrrolidon/Vinylacetat/ltaconsäure-Copolymere, Acrylsäure/Acrylamid-Copolymere bzw. Natriumsalze derselben vom Typ "Reten®"; etc.

Die erfindungsgemäß eingesetzetn Shampoos können selbstverständlich alle üblichen, in solchen Mitteln zum Einsatz gelangenden Stoffe enthalten.

Als solche seien beispielhaft Komplexbildner, Farbstoffe, Konservierungsmittel, pH-Regler, Viskositätsregler wie anorganische Salze, soweit sie nicht ohnehin in den Tensid-Ausgangsmischungen enthalten sind, Duftstoffe, Perlglanzmittel, Verdickungsmittel, Feuchthaltemittel, pflanzliche und tierische Öle, etc. genannt. Geeignete pflanzliche und tierische Öle sowie synthetische Fettsäureester, zu denen auch Wachse zählen, sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven-bzw. Sojaöl, Lanolin und dessen Derivate, ebenso Mineralöle wie Paraffinöl und Vaseline.

Synthetische Öle und Wachse sind beispielsweise Silikonöle, Polyethylengykole, etc. Weitere geeignete hydrophobe Komponenten sind insbesondere Fettalkohole, vorzugsweise solche mit etwa 8 bis 22 Kohlenstoffatomen im Molekül wie Myristyl-, Cetyl-, Stearylalkohol, Wachsalkohole und Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Polyethylenglykol- und Polyglycerylfettsäureester wie PEG-7-glycerylcocoat, Cetylpalmitat, etc.

Diese hydrophoben Komponenten sind in der erfindungsgemäß eingesetzten Zusammensetzungen vorzugsweise in einer Gesamtmenge von 0,5 bis 10, insbesondere 1 bis 7,5, vor allem 1,5 bis 5 Gew.-%, berechnet auf die Gesamtzusammensetzung, enthalten.

Eine Auflistung solcher Zusatzstoffe findet sich ebenfalls bei Schrader, I.c., auf S.695 bis 722.

Ein weiterer bevorzugter Bestandteil ist Ethoxydiglykol, vorzugsweise in einer Menge von 0,1 bis 5 Gew.-% des erfindungsgemäßen Shampoos.

Schließlich können auch noch bekannte Polysiloxane als konditionierende Mittel in den erfindungsgemäß eingesetzten flüssigen Haarwaschmitteln mitverwendet werden. Deren bevorzugter Anteil liegt dabei zwischen 0,5 und 5, insbesondere 1 bis 3 Gew.-% der Gesamtzusammensetzung. Geeignet sind sowohl leichtflüchtige als auch schwerflüchtige cyclische oder lineare Polysiloxane, beispielsweise die unter dem Trivialnamen "Dimethicone" bzw. "Phenyldimethicone" sowie "Cyclomethicone" bekannten Silikonöle.

Geeignet sind beispielsweise auch die in der EP-A 398 177 beschriebenen Silikonderivate, die dort in Kombination mit Alkylpolyglucosiden in flüssigen Detergens-Zusammensetzungen eingesetzt werden.

Gemäß einer bevorzugten Ausführungsform der Erfindung können erfindungsgemäße Shampoos auch Farbstoffe zur direkten oder oxidativen Färbung von Haaren enthalten, also sogenannte Tönungs- oder Färbeshampoos.
Besonders geeignet sind direktziehende Farbstoffe enthaltende Tönungsshampoos.

Es wurde nämlich festgestellt, daß solche Shampoos, die propoxylierte Fettsäurealkanolamide insbesondere kationische direktziehende Farbstoff enthalten, eine besonders brillante, glänzende und stabile Haarfärbung gegenüber bekannten Tönungsshampoos auf Basis konventioneller Shampoos ergeben.

Der pH-Wert der erfindungsgemäß eingesetzten Shampoos liegt im üblichen Bereich zwischen 5 und 8,5; für Spezialprodukte kann er auch unterhalb 5 eingestellt werden.

Die Viskosität liegt im üblichen Bereich zwischen 500 und 25000 mPa.s bei 20°C, vorzugsweise 1000 bis 15000, insbesondere 2500 bis 10000 mPa.s bei 20°C, gemessen nach Brookfield oder Höppler bei einer Scherspannung von 10 sec⁻¹.

Diese Zusammensetzungen können auch als Aerosol-Shampoos mit den üblichen Treibmitteln in Aluminiummonoblockdosen als auch Weißblechdosen, vorzugsweise mit Innenbeschichtung, z.B. auf Epoxyharz-Basis, versehen, als auch Kunststoffdosen abgepackt werden.
Die Herstellung der Produkte erfolgt durch Zusammenrühren der einzelnen Komponenten in Wasser, wobei auch Vormischungen verschiedener Bestandteile verwendet werden können.

Die folgenden Beispiele dienen der Illustration der Erfindung.

| Bestandteile | Nr. 1 | Nr. 2 | Nr. 3 | Nr. 4 |
|---|---|---|---|---|
| Natriumalkylethersulfat, (∼2,5 EO) | 8,5 | 10,0 | - | 8,0 |
| Natriumlauroylglutamat | - | - | - | 2,0 |
| Cocoamphoacetat | - | 3,0 | 2,0 | - |
| Cocoamidopropylbetain | 2,5 | - | 3,0 | - |
| Laureth-16 | - | - | 5,0 | - |
| C₁₂-C₁₄-Alkylpolyglucosid (P.D. ∼1,5) | 1,0 | 2,0 | 5,0 | 2,0 |
| PPG-2-Hydroxyethylcocamide (R=C₁₂-C₁₄, n=1) | 2,0 | 1,5 | 2,5 | 2,0 |
| Dinatrium-PEG-5 Lauroyl-Citrat-Sulfosuccinat | - | - | - | 3,0 |
| Polyquaternium-10 | 0,2 | 0,4 | - | - |
| Weizenproteinhydrolysat | 0,2 | - | - | 0,4 |
| Natriumchlorid | 0,5 | - | - | - |
| PEG-18-Glyceryloleat/cocoat | 2,0 | 0,5 | - | - |
| Parfum | 0,5 | - | 0,2 | - |
| Citronensäure | 0,4 | 0,5 | 0,5 | 0,5 |
| Panthenol | 0,5 | 0,5 | 0,3 | |
| Tocopherylacetat | 0,1 | 0,1 | 0,2 | |
| Konservierungsmittel | 0,2 | 0,2 | - | 0,2 |
| Wasser ad | 100,0 | 100,0 | 100,0 | 100,0 |

Diese Shampoo-Zusammensetzungen zeigten ein ausgezeichnetes Schaumvermögen (überraschenderweise auch die Zusammensetzung Nr. 3 , die kein anionisches Tensid enthielt) und ließen sich leicht auf dem Haar verteilen.
Bei dreimaliger Anwendung der Shampoos von jeweils 10 Personen traten keinerlei allergischen oder hautsensibilisierenden Effekte auf.
Überrraschenderweise ergab sich eine eindeutige Präferenz der Zusammensetzungen nach den Beispielen gegenüber gleichen Zusammensetzungen, die kein PPG-2 Hydroxylethylcocamide, sondern anstelle dessen die gleiche Menge Cocosmonoethanolamid enthielten, hinsichtlich des Glanzes, der Kämmbarkeit, der Glätte, der Lockerheit, der Sprungkraft und des Volumens der Haare im Doppelblindversuch an jeweils 10 Personen.

## Patentansprüche

1. Verwendung eines propoxylierten C₁₀-C₁₈-Fettsäurealkanolamids als haarkonditionierender Wirkstoff in Haarwaschmitteln, enthaltend 5 bis 50 Gew.-%, berechnet auf die Gesamtzusammensetzung, mindestens eines anionischen, nichtionischen, amphoteren und/oder zwitterionischen Tensids.

2. Verwendung eines Fettsäurealkanolamids der allgemeinen Formel 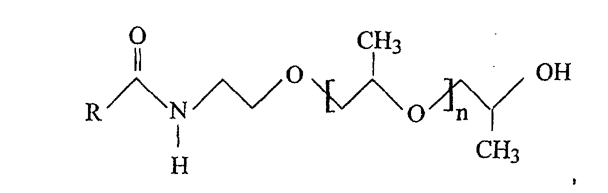 worin R eine C₁₀-C₁₈-Alkylgruppe und n 1 bis 3 bedeuten, nach Anspruch 1.

3. Verwendung von 0,25 bis 10 Gew.-%, berechnet auf die Gesamtzusammensetzung des Haarwaschmittels, eines Fettsäurealkanolamids nach Anspruch 1 und/oder 2.

## Claims

1. Use of a propoxylated C₁₀-C₁₈-fatty acid alkanolamide as hair-conditioning substance in shampoo compositions comprising 5 % to 50 % by weight, calculated to the total composition, of at least one anionic, nonionic, amphoteric and/or zwitterionic surfactant.

2. Use of a fatty acid alkanolamide of the general formula 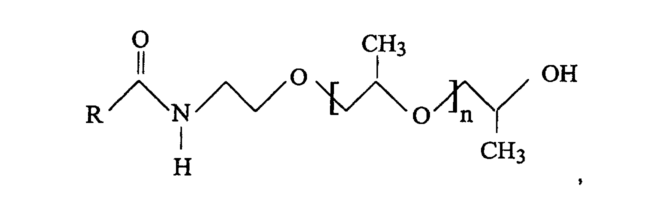 wherein R is a C₁₀-C₁₈-alkyl group and n is 1 to 3, according to claim 1.

3. Use of 0.25 % to 10 % by weight, calculated to the total shampoo composition, of a fatty acid alkanolamide according to claim 1 and/or 2.

## Revendications

1. Utilisation d'un alcanolamide d'acide gras en C₁₀ à C₁₈ propoxylé comme produit actif de conditionnement des cheveux dans des agents de lavage des cheveux qui contiennent de 5 à 50 % en poids, calculés par rapport à la composition totale, d'au moins un agent tensio-actif anionique, non ionique, amphotère et/ou zwitterionique.

2. Utilisation d'un alcanolamide d'acide gras selon la revendication 1, de formule générale 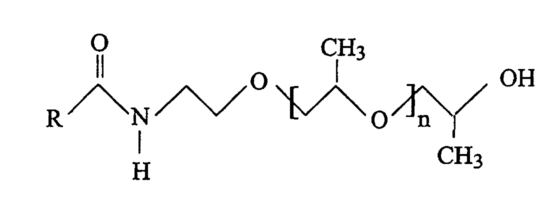 dans laquelle R représente un groupe alkyle en C₁₀ à C₁₈ et n un nombre de 1 à 3.

3. Utilisation de 0,25 à 10 % en poids, calculés par rapport à la composition totale de l'agent de lavage des cheveux, d'un alcanolamide d'acide gras selon les revendications 1 et/ou 2.
